# FASCICULE DE BREVET EUROPEEN

(11) **EP 3 624 758 B1**
(45) Date de publication et mention de la délivrance du brevet: **13.11.2024**
(21) Numéro de dépôt: 18735353.7
(22) Date de dépôt: 15.05.2018
(51) Int. Cl.: A61K 8/31, A61K 8/34, A61K 8/37, A61Q 5/00, A61Q 19/00, A61K 8/92, A61K 8/03

(54) **COMPOSITION COSMÉTIQUE BIPHASIQUE**
ZWEIPHASIGE KOSMETISCHE ZUSAMMENSETZUNG
BIPHASIC COSMETIC COMPOSITION

(30) Priorité: 19.05.2017 FR 1754433
(43) Date de publication de la demande: 25.03.2020
(73) Titulaire: LABORATOIRES M & L, 04100 Manosque (FR)
(72) Inventeur: LOCCI, Marie-Line, 04100 Manosque (FR); SBARRATO, Estelle, 13290 Les Milles (FR)
(74) Mandataire: Renard, Emmanuelle
(86) Numéro de dépôt international: PCT/FR2018/051171
(87) Numéro de publication internationale: WO 2018/211211

(56) Documents cités:
- WO-A1-2015/189340
- FR-A1- 2 208 642
- CLASSES DE SCIENCES AT HOME: "Miscibles, non miscibles ... y'a une bulle", October 2004 (2004-10-01), XP002774041, Retrieved from the Internet <URL:http://home.scarlet.be/at_home/miscible.htm> [retrieved on 20170919]

## Description

### OBJET DE L'INVENTION

La présente invention concerne une composition cosmétique comprenant une phase hydro-alcoolique et une phase huileuse particulières, permettant à la phase huileuse de former une inclusion sphérique ou ovoïde, d'aspect transparent ou translucide et aux contours nets, à l'intérieur de la phase hydro-alcoolique. Elle concerne également l'utilisation cosmétique de cette composition pour nourrir et/ou parfumer la peau et/ou les cheveux, ainsi qu'un procédé cosmétique utilisant cette composition.

### ARRIERE-PLAN DE L'INVENTION

Il existe sur le marché des compositions cosmétiques constituées de deux phases visuellement distinctes, utilisées en particulier comme lotions de démaquillage de la peau ou comme brumes de soin et/ou de protection des cheveux. Ces compositions biphasiques se distinguent des émulsions qui sont, au repos, constituées d'une dispersion, généralement non visible à l'oeil nu, de gouttelettes de phase aqueuse dispersées dans une phase huileuse, ou inversement. Dans les compositions biphasiques, la phase aqueuse est classiquement séparée de la phase huileuse par une seule interface continue, qui est généralement horizontale, la phase huileuse se trouvant en principe au-dessus de la phase aqueuse qui est plus dense. Un exemple d'une telle composition biphasique a été décrit par la Demanderesse dans la demande FR 3 038 514. Elle comprend une phase aqueuse glycérinée séparée d'une phase huileuse à base d'huiles végétales.

Il a toutefois été suggéré dans la demande WO 2015/189340 qu'il était possible de préparer des compositions de parfum biphasiques ayant un aspect différent au repos, l'une des phases formant une forme ovale ou sphérique à l'intérieur de l'autre, en ajustant les densités relatives des deux phases. La composition renferme de l'eau, de l'alcool, une huile apolaire (qui est un alcane ramifié) et un parfum. Une composition de ce type est décrite à l'Exemple 28. Elle renferme une phase parfumée et colorée, incluse dans une phase hydro-alcoolique. Néanmoins, l'Exemple 29 de WO 2015/189340 montre qu'en suivant l'enseignement de ce document, il n'est pas toujours possible de préparer une composition biphasique. A fortiori, ce document ne donne aucune indication à l'homme de l'art lui permettant de préparer une composition biphasique à base d'huile végétale. En effet, Il est à noter que les parfums sont généralement des huiles essentielles constituées majoritairement de terpénoïdes, qui sont des composés apolaires, contrairement aux triglycérides d'acides gras contenus dans les huiles végétales.

En outre, le document FR 2 208 642 enseigne qu'il est possible de disperser de l'huile sous forme d'une ou plusieurs sphères dans une phase hydroalcoolique en ajustant les tensions superficielles et les densités des deux phases. D'après ce document, toutes sortes d'huiles peuvent être utilisées, y compris des huiles végétales. Toutefois, l'homme de l'art n'est pas en mesure, sur la base des instructions générales contenues dans ce document, de préparer une inclusion d'huile végétale dans une phase hydroalcoolique.

Enfin, il est enseigné sur le site Internet *http:*//*home.scarlet.be*/*at_homelmiscible.htm* qu'il est possible de disperser de l'huile sous forme d'une ou plusieurs sphères dans une phase hydroalcoolique en ajustant les tensions superficielles et les densités des deux phases. Il n'est toutefois pas explicité comment une telle dispersion peut être obtenue dans le cas où on utilise une huile végétale.

Or, les compositions cosmétiques à base d'huiles végétales sont particulièrement recherchées par les consommateurs en raison de leurs propriétés émollientes et du caractère naturel de ces huiles, qui les différencie des huiles de synthèse ou d'origine pétrochimique, telles que la paraffine ou les silicones.

A la connaissance de la Demanderesse, il n'a donc jamais été proposé de composition biphasique renfermant une phase huileuse à base d'huile végétale formant une inclusion sphérique ou ovoïde à l'intérieur d'une phase aqueuse.

En outre, les compositions biphasiques nécessitent d'être agitées avant utilisation, afin de former un mélange extemporané homogène. Si ce mélange doit conserver une stabilité suffisante pendant toute l'application de la composition, les deux phases doivent se séparer ensuite rapidement au repos pour retrouver leur état initial.

Enfin, pour qu'une composition biphasique soit considérée comme commercialement viable, il est important qu'elle présente un aspect esthétique attrayant et, pour ce faire, que ses deux phases soient transparentes ou tout du moins translucides et séparées par une interface bien nette.

Après de nombreuses recherches, la Demanderesse est parvenue à concilier toutes ces exigences et à proposer une composition biphasique constituée d'une phase huileuse unique, comprenant une huile végétale et formant une inclusion sphérique ou ovoïde, d'aspect transparent ou translucide et aux contours nets, à l'intérieur d'une phase hydro-alcoolique. Ce besoin a pu être satisfait grâce à un choix judicieux des constituants des deux phases et de leurs proportions.

### RESUME DE L'INVENTION

L'invention a ainsi pour objet une composition cosmétique comprenant : (a) une phase hydro-alcoolique renfermant au moins 50% en poids d'alcool et (b) une phase huileuse renfermant un mélange d'au moins une huile végétale contenant un ou plusieurs esters d'acide gras insaturés, avec au moins une huile apolaire, étant entendu que :
- le rapport en poids entre l'huile apolaire et l'huile végétale est compris entre 0,75 et 2,
- la phase huileuse représente de 20 à 40% du poids total de la composition, et
- la phase hydro-alcoolique représente de 60 à 80% du poids total de la composition.

Elle a également pour objet l'utilisation cosmétique de cette composition pour nourrir et/ou parfumer la peau et/ou les cheveux.

Elle a encore pour objet un procédé cosmétique de soin et/ou de parfumage de la peau et/ou des cheveux, comprenant l'application topique sur la peau et/ou les cheveux de la composition précitée.

### BREVE DESCRIPTION DES FIGURES

La Figure 1 illustre une composition selon l'invention conditionnée dans un flacon équipé d'un pulvérisateur, après agitation (image de gauche) et au repos (image de droite).
La Figure 2 illustre une composition comparative renfermant un ratio huile apolaire / huile végétale inférieur à 0,75.
Les Figures 3 et 4 illustrent chacune une composition comparative dépourvue d'huile apolaire, conditionnée dans un flacon.

### DESCRIPTION DETAILLEE

A titre préliminaire, on notera que l'expression "compris entre" utilisée tout au long de cette description inclut les valeurs correspondant aux bornes inférieure et supérieure de l'intervalle concerné.

La composition selon l'invention comprend une phase hydro-alcoolique et une phase huileuse particulières, dont les constituants seront à présent détaillés.

### Phase huileuse

La phase huileuse de la composition selon l'invention représente de 20 à 40%, de préférence de 30 à 35%, du poids total de la composition.

Comme indiqué précédemment, elle contient au moins une huile végétale et au moins une huile apolaire.

Dans le contexte de cette description, on entend par "huile" un composé liquide à température ambiante (25°C) et pression atmosphérique (105 Pa) qui, lorsqu'il est introduit à raison d'au moins 1% en poids dans l'eau à 25°C, n'est pas du tout soluble dans l'eau, ou soluble à hauteur de moins de 10% en poids, par rapport au poids d'huile introduit dans l'eau.

Dans la suite de cette description, l'expression "une huile" ou "l'huile" désignera aussi bien l'unique huile, végétale ou apolaire, présente dans la composition qu'un mélange d'huiles végétales ou d'huiles apolaires, respectivement.

Les huiles végétales peuvent notamment être obtenues par pressage mécanique, souvent à froid, des fruits, noix ou graines de végétaux. Elles sont composées à environ 95 % de triglycérides, le reste étant des mono- et diglycérides et des composés dits insaponifiables tels que les alcools, caroténoïdes, la chlorophylle, les hydrocarbures, stérols et tocophérols. Les triglycérides peuvent eux-mêmes être simples (triesters de glycérol et de trois molécules du même acide gras) ou mixtes (triesters de glycérol et de deux ou trois acides gras différents), les esters mixtes étant majoritaires. Les acides gras contenus dans les huiles végétales sont saturés et/ou insaturés et leur longueur de chaîne est comprise entre 4 et 22 atomes de carbone et pour l'essentiel entre 12 et 18 atomes de carbone. On exprime généralement la teneur en acides gras des huiles après saponification, plutôt que leur teneur en triglycérides. Les acides gras majoritaires dans les huiles végétales sont les acides laurique (C12 saturé), palmitique (C16 saturé), stéarique (C18 saturé), palmitoléique (C16 mono-insaturé), oléique (C18 mono-insaturé), linoléique et linolénique (C18 polyinsaturés) et arachidonique (C20 polyinsaturés).

Des exemples d'huiles végétales contenant une proportion significative d'esters d'acides gras insaturés et précisément d'acides gras linéaires insaturés, sont notamment les huiles d'olive, de prune, de noisette, de soja, de tournesol, de tournesol oléique, de colza, de colza oléique, de noyaux d'abricot, d'arachide, de noix, de chanvre, de lin, de cameline, de germe de maïs, de pépins de raisin, de sésame et leurs mélanges. D'autres huiles végétales comprennent les huiles de germe de blé, d'argan, d'hibiscus, de coriandre, de ricin, de karité, d'avocat, d'amande douce, de coton, de macadamia, de jojoba, de luzerne, de pavot, de potimarron, de sésame, de courge, de cassis, d'onagre, de lavande, de bourrache, de millet, d'orge, de quinoa, de seigle, de carthame, de bancoulier, de passiflore, de rosier muscat, d'Echium ou de camélia, et leurs mélanges.

L'huile végétale peut préférentiellement renfermer des esters d'acides gras insaturés choisis parmi les mono-, di- et triesters d'au moins un acide gras insaturé comprenant l'acide oléique, l'acide linoléique et/ou l'acide linolénique, de préférence parmi les triglycérides d'au moins un acide gras insaturé comprenant l'acide oléique, l'acide linoléique et/ou l'acide linolénique, telle qu'une huile de tournesol, de tournesol oléique, de colza, de colza oléique, de noisette ou d'olive ou leurs mélanges. On préfère selon l'invention utiliser une huile végétale renfermant au moins 60% en poids d'esters d'acide oléique et qui peut par ailleurs contenir d'autres esters d'acides gras insaturés tels que définis ci-dessus, en particulier une huile de tournesol oléique, de colza oléique, de noisette ou d'olive et, mieux, une huile de tournesol oléique.

L'huile de tournesol est typiquement riche en esters d'acide linoléique (plus de 60% en poids), qui est un acide gras polyinsaturé de type ω-6, tandis que l'huile de tournesol oléique est obtenue à partir d'un tournesol sélectionné de telle manière que son huile renferme majoritairement (généralement plus de 80% en poids) des esters d'acide oléique, qui est un acide gras mono-insaturé de type ω-9.

En plus de l'huile précitée, qui est par nature polaire, la composition selon l'invention renferme au moins une huile apolaire. Par "huile apolaire", on entend au sens de cette description une huile ayant une tension interfaciale supérieure à 30 mN/m, telle que mesurée suivant la norme ASTM D971-99a (2004).

Des exemples d'huiles apolaires sont les dialkyléthers tels que le dicaprylyl éther ; les dialkylcarbonates tels que le dicaprylyl carbonate ; les hydrocarbures tels que le squalane et le squalène, les polyoléfines telles que le polyisobutène hydrogéné et le polydécène, les huiles minérales, les isoparaffines en C₁₂-C₁₆, notamment l'isododécane et l'isohexadécane, et les alcanes linéaires en C₁₀-C₂₀ ; les esters d'acides en C₁₂-C₄₄ tels que le laurate d'hexyle ; et leurs mélanges.

Selon une forme d'exécution préférée de l'invention, les huiles apolaires comprennent ou sont constituées d'au moins une huile hydrocarbonée, constituée d'un alcane linéaire ou ramifié en C₁₀-C₂₀. Certains de ces alcanes sont volatils, notamment le décane, le undécane et l'isododécane, et d'autres ne sont pas volatils, notamment le tétradécane et le pentadécane. Par "alcane volatil", on entend un alcane ayant une pression de vapeur de l'ordre de 0,001 à 300 mm Hg et de préférence de 0,01 à 10 mm Hg à température ambiante (20°C) et pression atmosphérique. Avantageusement, ces alcanes présentent en outre un point éclair (mesuré selon la norme ASTM D93) inférieur à 100°C, de préférence compris entre 50 et 95°C, par exemple entre 70 et 90°C, voire entre 80 et 90°C et/ou une viscosité cinématique inférieure à 5 cSt, voire comprise entre 1 et 3 cSt, à 40°C.

Des exemples d'alcanes linéaires sont les alcanes linéaires en C₁₁ et C₁₃ tels que décrits notamment dans la demande EP 1798213 et commercialisés par la société BASF sous la dénomination commerciale Cetiol^{®} Ultimate^{®} ; le mélange d'alcanes linéaires en C₁₂ et C₁₄ commercialisé par la société BIOSYNTHIS sous la dénomination commerciale Vegelight^{®} ; les alcanes linéaires disponibles auprès de la société SASOL sous la dénomination commerciale Parafol^{®} ; et l'alcane linéaire en C₁₅ disponible auprès de la société AMYRIS sous la dénomination commerciale Neossance Hemisqualane^{®}.

On préfère selon l'invention utiliser le dodécane, l'isododécane, le pentadécane, l'isohexane et leurs mélanges et plus préférentiellement le pentadécane. Selon une forme d'exécution particulièrement préférée de l'invention, le pentadécane mis en oeuvre dans la composition peut être d'origine végétale, notamment produit par fermentation microbienne de sucres.

Le rapport en poids de l'huile apolaire à l'huile végétale est compris entre 0,75 et 2, préférentiellement entre 0,8 et 1,5 et plus préférentiellement entre 0,9 et 1,2.

Selon un mode de réalisation avantageux de l'invention, la phase huileuse renferme au moins un parfum et/ou au moins un agent anti-oxydant tel que le tocophérol et ses esters.

On préfère que la phase huileuse selon l'invention ne renferme pas de composé siliconé. Par ailleurs, on préfère que la phase huileuse ne contienne pas d'autre constituant que les huiles végétale(s) et apolaire(s) précitées et éventuellement un ou plusieurs parfums, colorants, anti-oxydants, conservateurs ou filtres solaires liposolubles ou lipodispersibles. En particulier, la phase huileuse ne renferme généralement pas de gélifiant de phase grasse ni d'émulsionnant.

Selon un mode de réalisation préféré de l'invention, l'huile hydrocarbonée et l'huile apolaire représentent ensemble de 80 à 100%, par exemple de 90 à 100%, mieux, de 95 à 100% du poids total de la phase huileuse.

### Phase hydro-alcoolique

La phase hydro-alcoolique de la composition selon l'invention renferme de l'eau et au moins un alcool en C₂-C₄, de préférence l'éthanol ou l'isopropanol, plus préférentiellement l'éthanol. Lorsqu'il est utilisé dans la présente invention, l'éthanol a de préférence un degré alcoolique supérieur à 80%, plus préférentiellement supérieur à 85%, mieux, supérieur à 90% voire à 95%.

L'alcool représente au moins 50% en poids, par rapport au poids de la phase hydro-alcoolique. De son côté, la phase hydro-alcoolique représente de 60 à 80%, préférentiellement de 60 à 75% et, mieux, de 65 à 70% du poids total de la composition.

Le rapport en poids de l'alcool à l'eau est avantageusement compris entre 5:1 et 10:1 préférentiellement entre 7:1 et 9:1, plus préférentiellement entre 7,5:1 et 8,5:1.

La phase hydro-alcoolique de la composition selon l'invention est avantageusement exempte de gélifiant hydrophile et/ou de polyol et/ou de tensioactif et/ou de sels tels que le bicarbonate de sodium et/ou le bromure d'ammonium. Selon une forme d'exécution préférée de l'invention, elle est exempte de tous ces constituants. En revanche, elle peut éventuellement inclure un ou plusieurs actifs hydrophiles tels que des sucres (éventuellement sous forme de miel), des acides aminés, des eaux florales, des extraits végétaux, de la vitamine B5 et de la glycérine ; des conservateurs ; des chélatants ; des parfums ; des filtres UV hydrosolubles ; des colorants ; des ajusteurs de pH ; et leurs mélanges.

On préfère par ailleurs que la composition selon l'invention soit exempte de charges pulvérulentes et de pigments.

Selon un mode de réalisation préféré de l'invention, l'alcool et l'eau représentent ensemble de 80 à 100%, par exemple de 90 à 100%, mieux, de 95 à 100% du poids total de la phase hydro-alcoolique.

Les phases hydro-alcoolique et huileuse décrites précédemment peuvent être mélangées de manière classique pour l'homme du métier, après homogénéisation de leurs constituants respectifs, pour obtenir la composition selon l'invention. Une fois la composition constituée et placée dans un conditionnement tel qu'un flacon, elle déphase rapidement, après quelques secondes, de telle manière que la phase huileuse forme une inclusion sphérique ou ovoïde (ou "bulle") à l'intérieur de la phase hydro-alcoolique. La position de cette bulle dans le flacon contenant la composition selon l'invention dépend de la densité relative des deux phases, qui peut être ajustée en modifiant la nature de l'huile apolaire, le ratio alcool / eau de la phase hydro-alcoolique et/ou le ratio huile apolaire / huile végétale de la phase huileuse. En outre, les dimensions de la bulle peuvent être ajustées en modifiant le rapport pondéral de la phase huileuse à la phase hydro-alcoolique.

La composition décrite précédemment peut être utilisée pour le soin et/ou le parfumage de la peau et/ou des cheveux, notamment pour les nourrir. Cette composition peut en particulier être appliquée sur l'ensemble du corps, éventuellement à l'exclusion du visage. La composition peut par exemple être appliquée sur la peau et/ou les cheveux une à trois fois par jour, par exemple matin et/ou soir. Elle peut avantageusement être conditionnée dans un flacon pourvu d'un pulvérisateur. Il s'agit généralement d'une composition non rincée.

### EXEMPLES

L'invention sera mieux comprise à la lumière des exemples suivants, qui sont donnés à titre purement illustratif et n'ont pas pour but de limiter la portée de l'invention, définie par les revendications annexées.

### Exemple 1 : Composition biphasique

On a mélangé les ingrédients mentionnés ci-dessous dans les proportions pondérales également indiquées ci-dessous.

### Phase huileuse

| | |
|---|---|
| Huiles végétales⁽¹⁾ | 15,5 % |
| Pentadécane | 15,5 % |
| Conservateurs | qs |
| Parfum | qs |

### Phase aqueuse

| | |
|---|---|
| Eau | 7,5 % |
| Ethanol | 59,0 % |
| Total | 100,0 % |

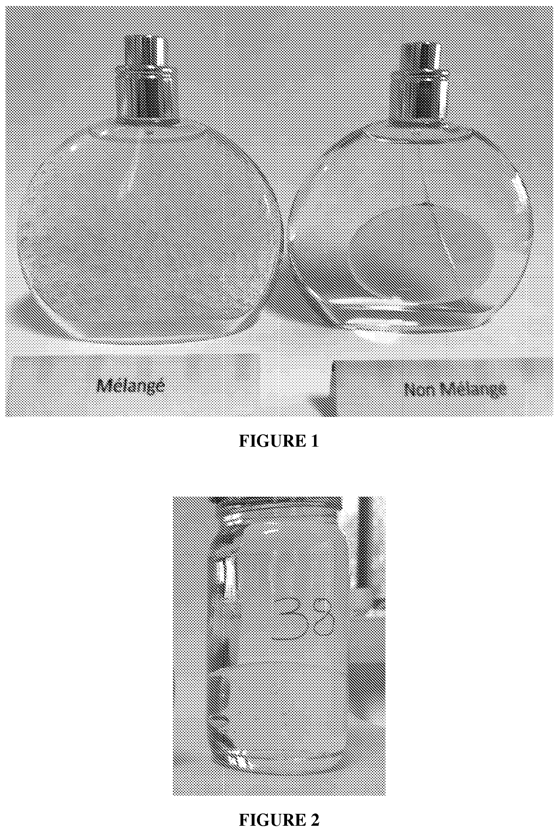

Cette composition a été préparée de la manière suivante : les phases aqueuse et huileuse sont préparées séparément, par mélange sous agitation de leurs constituants respectifs. La phase huileuse, puis la phase aqueuse, sont ensuite introduites dans un flacon qui est alors équipé d'un pulvérisateur pour obtenir le produit illustré à la Figure 1. Comme il ressort de cette Figure, la composition se présente, au repos, sous une forme biphasique comprenant une grosse bulle transparente (constituée de la phase huileuse) incluse dans un milieu continu (la phase hydro-alcoolique). Après une brève agitation, elle prend la forme d'une émulsion constituée de grosses gouttelettes dispersées de manière homogène dans le milieu.

### Exemple 2 : Essais comparatifs

On a préparé plusieurs compositions similaires à celles de l'Exemple 1 en faisant varier la nature et/ou la proportion de ses constituants. L'aspect des compositions obtenues a été évalué visuellement. Les résultats de ces essais sont rassemblés dans le Tableau 1 ci-après.

**Tableau 1**

| | **1** | **2** | **3** | **4** | **5** | **6** | **7** | **8** | **9** | **10** | **11** | **12** |
|---|---|---|---|---|---|---|---|---|---|---|---|---|
| Eau (%) | 8 | 8 | 7 | 7,5 | 7,5 | 7,5 | 9 | 19 | 12 | 30 | 7 | 9 |
| Alcool (%) | 61 | 61 | 62 | 59,5 | 59,5 | 59,5 | 58 | 50 | 62 | 28 | 62 | 60 |
| Ratio alcool/eau | 7,6 | 7,6 | 8,9 | 7,9 | 7,9 | 7,9 | 6,4 | 2,6 | 5,2 | 0,93 | 8,9 | 6,7 |
| Huile végétale (%) | 15 | 17 | 15 | 15 | 15 | 15 | 15 | 31 | 30 | 31 | 18 | 18 |
| Alcane (%) Type* | 16 C15L | 14 C15L | 16 C15L | 15 C15L | 15 C12R | 15 C16 R | 16 C15L | 0 | 0 | 0 | 13 | 13 |
| Ratio alcane/ huile végétale | 1,1 | 0,82 | 1,1 | 1 | 1 | 1 | 1,1 | -- | -- | -- | 0,72 | 0,72 |
| Observations | | | | | | | | | | | | |
| Bulle | oui | oui | oui | oui | oui | oui | oui | dôme | non | dôme | non | non |
| Position | bas | bas | | bas | haut | bas | haut | haut | | bas | | bas |
| Interface | nette | nette | nette | nette | nette | nette | nette | floue | | floue | | |

| | | | | | | | | | | | | |
|---|---|---|---|---|---|---|---|---|---|---|---|---|
| * Nombre d'atomes de carbone, L = linéaire ; R = ramifié | | | | | | | | | | | | |

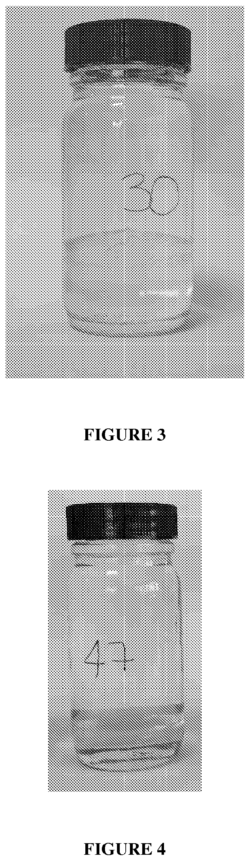

Comme il ressort de ce tableau (Formules n° 8 à 10), la présence d'une huile apolaire est nécessaire à l'obtention d'une bulle de contours nets. Les Figures 3 et 4 illustrent les compositions obtenues à partir des Formules 10 et 9, respectivement. En outre, comme illustré par la Formule n° 12 et la Figure 2 correspondante, cette huile apolaire doit représenter une quantité supérieure à 72% du poids de l'huile végétale. Enfin, la comparaison des Formules n° 7 et 4 montre qu'il est possible d'ajuster la position de la bulle dans le flacon en modifiant le ratio éthanol / eau. Elle peut en variante être ajustée en modifiant la nature de l'alcane (Formules n° 4, 5 et 6).

## Revendications

1. Composition cosmétique comprenant : (a) une phase hydro-alcoolique renfermant au moins 50% en poids d'alcool et (b) une phase huileuse renfermant un mélange d'au moins une huile végétale contenant un ou plusieurs esters d'acide gras insaturés, avec au moins une huile apolaire, étant entendu que :
- le rapport en poids entre l'huile apolaire et l'huile végétale est compris entre 0,75 et 2,
- la phase huileuse représente de 20 à 40% du poids total de la composition, et
- la phase hydro-alcoolique représente de 60 à 80% du poids total de la composition.

2. Composition selon la revendication 1, **caractérisée en ce que** l'ester d'acide gras insaturé est choisi parmi les mono-, di- et triesters d'au moins un acide gras insaturé comprenant l'acide oléique, l'acide linoléique et/ou l'acide linolénique, de préférence parmi les triglycérides d'au moins un acide gras insaturé comprenant l'acide oléique, l'acide linoléique et/ou l'acide linolénique.

3. Composition selon la revendication 1 ou 2, **caractérisée en ce que** l'huile végétale est choisie parmi les huiles d'olive, de prune, de noisette, de soja, de tournesol, de tournesol oléique, de colza, de colza oléique, de noyaux d'abricot, d'arachide, de noix, de chanvre, de lin, de cameline, de germe de maïs, de pépins de raisin, de sésame et leurs mélanges, préférentiellement l'huile végétale est une huile de tournesol, de tournesol oléique, de colza, de colza oléique, de noisette ou d'olive ou leurs mélanges, mieux, l'huile végétale est une huile de tournesol oléique.

4. Composition selon l'une quelconque des revendications 1 à 3, **caractérisée en ce que** l'huile apolaire est une huile hydrocarbonée choisie parmi les alcanes linéaires ou ramifiés en C₁₀-C₂₀, tels que le dodécane, l'isododécane, le pentadécane, l'isohexane et leurs mélanges et est de préférence constituée du pentadécane.

5. Composition selon l'une quelconque des revendications 1 à 4, **caractérisée en ce que** le rapport en poids de l'huile apolaire à l'huile végétale est compris entre 0,8 et 1,5 et plus préférentiellement entre 0,9 et 1,2.

6. Composition selon l'une quelconque des revendications 1 à 5, **caractérisée en ce que** la phase huileuse représente de 30 à 35% du poids total de la composition.

7. Composition selon l'une quelconque des revendications 1 à 6, **caractérisée en ce que** la phase hydro-alcoolique représente de 60 à 75% et, mieux, de 65 à 70% du poids total de la composition.

8. Composition selon l'une quelconque des revendications 1 à 7, **caractérisée en ce que** la phase hydroalcoolique renferme de l'eau et au moins un monoalcool en C₂-C₄, de préférence l'éthanol ou l'isopropanol, plus préférentiellement l'éthanol.

9. Composition selon la revendication 8, **caractérisée en ce que** le rapport en poids de l'alcool à l'eau est compris entre 5:1 et 10:1, préférentiellement entre 7:1 et 9:1 et plus préférentiellement entre 7,5:1 et 8,5:1.

10. Utilisation cosmétique de la composition selon l'une quelconque des revendications 1 à 9 pour nourrir et/ou parfumer la peau et/ou les cheveux.

11. Procédé cosmétique de soin et/ou de parfumage de la peau et/ou des cheveux, comprenant l'application topique sur la peau et/ou les cheveux d'une composition selon l'une quelconque des revendications 1 à 9.

## Patentansprüche

1. Kosmetikzusammensetzung, umfassend: (a) eine wässrig-alkoholische Phase, die mindestens 50 Gew.-% Alkohol enthält, und (b) eine Ölphase, die ein Gemisch von mindestens einem Pflanzenöl, das einen oder mehrere ungesättigte Fettsäureester enthält, mit mindestens einem unpolaren Öl enthält, mit der Maßgabe, dass:
- das Gewichtsverhältnis zwischen dem unpolaren Öl und dem Pflanzenöl zwischen 0,75 und 2 beträgt,
- die Ölphase 20 bis 40 Gew.-%, bezogen auf das Gesamtgewicht der Zusammensetzung, ausmacht und
- die wässrig-alkoholische Phase 60 bis 80 Gew.-%, bezogen auf das Gesamtgewicht der Zusammensetzung, ausmacht.

2. Zusammensetzung nach Anspruch 1, **dadurch gekennzeichnet, dass** der ungesättigte Fettsäureester aus Mono-, Di- und Triestern mindestens einer ungesättigten Fettsäure, umfassend Ölsäure, Linolsäure und/oder Linolensäure, vorzugsweise aus Triglyceriden mindestens einer ungesättigten Fettsäure, umfassend Ölsäure, Linolsäure und/oder Linolensäure, ausgewählt ist.

3. Zusammensetzung nach Anspruch 1 oder 2, **dadurch gekennzeichnet, dass** das Pflanzenöl aus Oliven-, Pflaumen-, Haselnuss-, Soja-, Sonnenblumen-, Ölsäuresonnenblumen-, Raps-, Ölsäureraps-, Aprikosenkern-, Erdnuss-, Nuss-, Hanf-, Lein-, Leindotter-, Maiskeim-, Traubenkern-, Sesamöl und deren Gemischen ausgewählt ist, vorzugsweise ist das Pflanzenöl ein Sonnenblumen-, Ölsäuresonnenblumen-, Raps-, Ölsäureraps-, Haselnuss- oder Olivenöl oder deren Gemische, besser ist das Pflanzenöl ein Ölsäuresonnenblumenöl.

4. Zusammensetzung nach einem der Ansprüche 1 bis 3, **dadurch gekennzeichnet, dass** das unpolare Öl ein Kohlenwasserstofföl ist, das aus geraden oder verzweigten C₁₀-C₂₀-Alkanen, wie Dodecan, Isododecan, Pentadecan, Isohexan, und deren Gemischen ausgewählt ist und vorzugsweise aus Pentadecan besteht.

5. Zusammensetzung nach einem der Ansprüche 1 bis 4, **dadurch gekennzeichnet, dass** das Gewichtsverhältnis von unpolarem Öl zu Pflanzenöl zwischen 0,8 und 1,5 und stärker bevorzugt zwischen 0,9 und 1,2 beträgt.

6. Zusammensetzung nach einem der Ansprüche 1 bis 5, **dadurch gekennzeichnet, dass** die Ölphase 30 bis 35 Gew.-%, bezogen auf das Gesamtgewicht der Zusammensetzung, ausmacht.

7. Zusammensetzung nach einem der Ansprüche 1 bis 6, **dadurch gekennzeichnet, dass** die wässrig-alkoholische Phase 60 bis 75 Gew.-% und besser 65 bis 70 Gew.-%, bezogen auf das Gesamtgewicht der Zusammensetzung, ausmacht.

8. Zusammensetzung nach einem der Ansprüche 1 bis 7, **dadurch gekennzeichnet, dass** die wässrig-alkoholische Phase Wasser und mindestens einen C₂-C₄-Monoalkohol, vorzugsweise Ethanol oder Isopropanol, stärker bevorzugt Ethanol, enthält.

9. Zusammensetzung nach Anspruch 8, **dadurch gekennzeichnet, dass** das Gewichtsverhältnis von Alkohol zu Wasser zwischen 5:1 und 10:1, vorzugsweise zwischen 7:1 und 9:1 und stärker bevorzugt zwischen 7,5:1 und 8,5:1 beträgt.

10. Kosmetische Verwendung der Zusammensetzung nach einem der Ansprüche 1 bis 9 zum Nähren und/oder Parfümieren der Haut und/oder der Haare.

11. Kosmetikverfahren zur Pflege und/oder zum Parfümieren der Haut und/oder der Haare, umfassend das topische Aufbringen einer Zusammensetzung nach einem der Ansprüche 1 bis 9 auf die Haut und/oder die Haare.

## Claims

1. A cosmetic composition comprising: (a) an aqueous-alcoholic phase containing at least 50% by weight of alcohol and (b) an oily phase containing a mixture of at least one plant oil containing one or more unsaturated fatty acid esters, with at least one nonpolar oil, with the proviso that:
- the weight ratio between the nonpolar oil and the plant oil is between 0.75 and 2,
- the oily phase represents from 20 to 40% of the total weight of the composition,
- the aqueous-alcoholic phase represents from 60 to 80% of the total weight of the composition.

2. The composition as claimed in claim 1, **characterized in that** the unsaturated fatty acid ester is chosen from mono-, di- and triesters of at least one unsaturated fatty acid comprising oleic acid, linoleic acid and/or linolenic acid, preferably from triglycerides of at least one unsaturated fatty acid comprising oleic acid, linoleic acid and/or linolenic acid.

3. The composition as claimed in claim 1 or 2, **characterized in that** the plant oil is chosen from olive oil, plum oil, hazelnut oil, soybean oil, sunflower oil, oleic sunflower oil, rapeseed oil, oleic rapeseed oil, apricot kernel oil, groundnut oil, walnut oil, hemp oil, linseed oil, camelina oil, corn germ oil, grapeseed oil, sesame oil and mixtures thereof, preferentially the plant oil is a sunflower oil, oleic sunflower oil, rapeseed oil, oleic rapeseed oil, hazelnut oil or olive oil or mixtures thereof, better still the plant oil is an oleic sunflower oil.

4. The composition as claimed in any one of claims 1 to 3, **characterized in that** the nonpolar oil is a hydrocarbon-based oil chosen from linear or branched C₁₀-C₂₀ alkanes, such as dodecane, isododecane, pentadecane, isohexane and mixtures thereof and preferably consists of pentadecane.

5. The composition as claimed in any one of claims 1 to 4, **characterized in that** the weight ratio of the nonpolar oil to the plant oil is between 0.8 and 1.5 and more preferentially between 0.9 and 1.2.

6. The composition as claimed in any one of claims 1 to 5, **characterized in that** the oily phase represents from 30 to 35% of the total weight of the composition.

7. The composition as claimed in any one of claims 1 to 6, **characterized in that** the aqueous-alcoholic phase represents from 60 to 75% and better still from 65 to 70% of the total weight of the composition.

8. The composition as claimed in any one of claims 1 to 7, **characterized in that** the aqueous-alcoholic phase contains water and at least one C₂-C₄ monoalcohol, preferably ethanol or isopropanol, more preferentially ethanol.

9. The composition as claimed in claim 8, **characterized in that** the weight ratio of the alcohol to the water is between 5:1 and 10:1, preferentially between 7:1 and 9:1 and more preferentially between 7.5:1 and 8.5:1.

10. The cosmetic use of the composition as claimed in any one of claims 1 to 9, for nourishing and/or fragrancing the skin and/or the hair.

11. A cosmetic process for caring for and/or fragrancing the skin and/or the hair, comprising the topical application of a composition as claimed in any one of claims 1 to 9 to the skin and/or the hair.
